# EUROPEAN PATENT SPECIFICATION

(11) **EP 0 629 400 B1**
(45) Date of publication and mention of the grant of the patent: **22.09.1999**
(21) Application number: 94303847.1
(22) Date of filing: 27.05.1994
(51) Int. Cl.: A61K 31/12, A61K 9/20

(54) **Idebenone compositions for treating Alzheimer's disease**
Idebenone-haltige Zusammensetzungen zur Behandlung von M. Alzheimer
Compositions contenant de l'idebenone pour le traitement de la maladie d'Alzheimer

(30) Priority: 18.06.1993 JP 14815493
(43) Date of publication of application: 21.12.1994
(73) Proprietor: TAKEDA CHEMICAL INDUSTRIES, LTD., Chuo-ku, Osaka 541 (JP)
(72) Inventor: Miyamoto, Masaomi, Takarazuka, Hyogo 665 (JP); Nagaoka, Akinobu, Kawanishi, Hyogo 666-01 (JP); Goto, Giichi, Toyono-gun, Osaka 563-01 (JP)
(74) Representative: Hall, Marina

(56) References cited:
- US-A- 5 059 627
- ARCH. GERONT. GERIATR. vol. 15 , 1992 pages 249 - 260 U. SENIN ET AL., 'Idebenone in senile dementia of Alzheimer type: A multicentre study'
- ARZNEIM.-FORSCH./DRUG RES. vol. 35, no. II , 1985 pages 1704 - 1707 BARKWORTH ET AL., 'An early phase I study to determine the tolerance, safety and pharmacokinetics of Idebenone following multiple oral doses'
- ARCH. GERONTOL. GERIATR. vol. 8 , 1989 pages 355 - 366 T. NAKANO ET AL., 'Effects of idebenone on electroencephalograms of patients with cerebrovascular disorders'
- J. AICHI MED. UNIV. ASSOC. vol. 17, no. 6 , 1989 pages 775 - 782 K. SAHASHI ET AL. 'a newly recognised therapeutic basis of idebenone for mitochondrial encephalomyopathy'
- CLINICAL HEMORHEOLOGY vol. 11 , 1991 pages 351 - 359 Y. NAGAKAWA ET AL. 'Effect of idebenone on hemorheologic variables in geriatric patients with cerebral infarction'
- Rosen et al.; Am. J. Psychiatry, 1984, 141:1356-1364
- Davis et al.; N. Engl. J. Med., 1992, 327(18): 1253-1259
- Knapp et al.; JAMA, 6.4.1994, 271(13):985-991

## Description

The present invention relates to the treatment of Alzheimer's disease, a cause of dementia.

The incidence of dementia is increasing in the ageing society. Alzheimer's disease, in particular, is of major concern, calling for the elucidation of its cause and the development of a therapy.

The specification of U.S. Patent No. 5059627 describes that idebenone is effective in the treatment of Alzheimer's disease when administered at daily doses of 0.1 to 500 mg in adults. That specification, however, presents no specific pharmacological data which demonstrate the effect, except for the induction and promotion of nerve growth factor (NGF) secretion observed using mouse astroglias, and presents no clinical data on idebenone administration to patients with Alzheimer's disease.

Also, on pages 249-260 of Arch. Gerontol, Geriatr., Vol. 15, (1992), it is stated that idebenone proved effective in patients with Alzheimer's disease when administered at 45 mg twice a day (daily dose 90 mg). That publication, however, describes no cases where idebenone was administered at doses exceeding 50 mg per administration or 90 mg per day.

It is generally held that the efficacy of pharmaceuticals does not improve when the dose is increased above conventional doses because the toxicity increases. Despite this, the present inventors found that a more excellent effect than expected can be obtained by administering idebenone to patients with Alzheimer's disease at 50 mg or more per administration or 150 mg or more per day, high doses not used conventionally. The inventors conducted investigations based on this finding, and developed the present invention.

Accordingly, the present invention provides a use of idebenone for the manufacture of a medicament for oral administration for the treatment of Alzheimer's disease comprising a unit dosage of 90 mg or more of idebenone, wherein the amount of idebenone for administration per day is 270 mg.

The invention also provides a use of idebenone for the manufacture of a medicament for oral administration for the treatment of Alzheimer's disease comprising a unit dosage of 90 mg or more of idebenone, wherein the amount of idebenone for administration per day is 360 mg.

The invention also provides a use of idebenone for the manufacture of a medicament for oral administration for the treatment of Alzheimer's disease comprising a unit dosage of 90 mg or more of idebenone, wherein the amount of idebenone for administration per day is 450 mg.

Idebenone as used in the present invention is described in the specification for Japanese Patent Examined Publication JP-B-62003134 (1987, filed by the present applicant), its chemical name being 6-(10-hydroxydecyl)-2,3-dimethoxy-5-methyl-1,4-benzoquinone.

To patients with Alzheimer's disease, idebenone can be orally administered in various dosage forms as pharmaceutical compositions prepared by known methods, such as those described in the specifications for Japanese Patent Examined Publication Nos. JP-B-1012727 (1989), JP-B-63051123 (1988) and JP-B-1039405 (1989) and Japanese Patent Unexamined Publication No. JP-A-3081218 (1991) all filed by the present applicant. The composition for oral administration such as powder, granule, tablet, hard capsule and soft capsule may be prepared by a per se known conventional manner, and may comprise carriers, excipients or diluents conventionally used in the pharmaceutical art. For example, suitable carriers or excipients include lactose, starch, sugar, magnesium stearate, etc. As the excipients in the preparation of soft capsules, there may be used nontoxic, pharmaceutically acceptable oils and fats of animal, vegetable or mineral origin. The essential active ingredients are generally dissolved in these oils and fats before filing soft capsules therewith. Although any dosage form is acceptable, including tablets, fine subtilaes, granules and capsules, tablets, fine subtilaes and capsules are preferred, with greater preference given to tablets and fine subtilaes. These may be prepared as sustained-release preparations as necessary. A sustained-release preparation may be prepared as tablets, granules, fine subtilaes or capsules by known methods. A sustained-release preparation can be obtained by coating tablets, granules, fine subtilaes or capsules with oils or fats (triglycerides), fatty acid esters of polyglycerol, hydroxypropyl cellulose etc. by a conventional method.

Tablet or capsule containing idebenone in an amount of 90 mg or more is preferable, and granules or fine subtilaes are preferably packed so that one dose of granules or fine subtilaes contain idebenone in an amount of 90 mg or more.

The toxicity of idebenone is very low; for example, its LD₅₀ as an index of acute toxicity exceeds 10,000 mg/kg in male and female mice, exceeds 10,000 mg/kg in male rats and is about 10,000 mg/kg in female rats.

Although doses of idebenone vary depending on symptoms and other factors, the usual adult dose for oral administration is 450 mg per day, preferably 360 mg per day, and more preferably 270 mg per day.

The daily dose of idebenone may be administered in a number of portions, usually 3 to 5 portions daily, preferably 3 portions. The dose per administration is 90 mg or more. It is recommended that when idebenone is administered in 3 to 5 portions a day, there be an interval of at least 4 hours between administrations, and when it is administered in 2 portions a day, an interval of at least 6 hours. Although timing of administration is not subject to limitation, it is preferable that idebenone be administered after meals.

Idebenone is more effective in patients with severe symptoms of Alzheimer's disease than in those with mild symptoms.

### [Examples]

### Example 1

| 90 mg tablets (per tablet) | |
|---|---|
| Idebenone | 90.000 mg |
| Lactose, EP | 233.186 mg |
| α-converted starch | 11.210 mg |
| Calcium salt of carboxymethyl cellulose (ECG 505) | 67.270 mg |
| Magnesium stearate, EP | 1.120 mg |
| Hydroxypropylmethyl cellulose, USP (Pharmacoat 606) | 5.573 mg |
| Polyethylene glycol NF, 6000 | 1.393 mg |
| Propylene glycol, EP | 0.465 mg |
| Talc, EP | 1.858 mg |
| Titanium oxide, EP, E171 | 2.786 mg |
| Red Color 30, E172 | 0.139 mg |
| Total | 415.000 mg |

### Comparative Example 1

| 30 mg tablets (per tablet) | |
|---|---|
| Idebenone | 30.000 mg |
| Lactose, EP | 293.186 mg |
| α-converted starch | 11.210 mg |
| Calcium salt of carboxymethyl cellulose (ECG 505) | 67.270 mg |
| Magnesium stearate, EP | 1.120 mg |
| Hydroxypropylmethyl cellulose, USP (Pharmacoat 606) | 5.573 mg |
| Polyethylene glycol NF, 6000 | 1.393 mg |
| Propylene glycol, EP | 0.465 mg |
| Talc, EP | 1.858 mg |
| Titanium oxide, EP, E171 | 2.786 mg |
| Red Color 30, E172 | 0.139 mg |
| Total | 415.000 mg |

### Comparative Example 2

| Placebo tablets (per tablet) | |
|---|---|
| Lactose, EP | 274.116 mg |
| Corn starch | 112.630 mg |
| Hydroxypropyl cellulose | 12.230 mg |
| L-orange 2, E110 | 0.360 mg |
| Yellow quinoline 70, E194 | 2.340 mg |
| Magnesium stearate, EP | 1.110 mg |
| Hydroxypropylmethyl cellulose, USP (Pharmacoat 606) | 5.573 mg |
| Polyethylene glycol NF, 6000 | 1.393 mg |
| Propylene glycol, EP | 0.465 mg |
| Talc, EP | 1.858 mg |
| Titanium oxide, EP, E171 | 2.786 mg |
| Red Color 30, E172 | 0.139 mg |
| Total | 415.000 mg |

### Example 2

| | |
|---|---|
| (1) Idebenone | 90 g |
| (2) Lactose | 102 g |
| (3) Corn starch | 40 g |
| (4) Magnesium stearate | 2 g |
| (5) Crystalline cellulose | 26 g |

The whole amounts of ingredients (1), (2) and (5) and 15 g of corn starch (3) were made into pasto and granulated. To these granules, the remaining portion of corn starch and ingredient (4) were added, and the mixture was compressed using a compressive tableting machine to yield 1,000 tablets of 5 mm diameter containing 90 mg of idebenone per tablet.

### Experimental Example

A double-blind controlled study was conducted in patients with Alzheimer type dementia.
- Subjects: : Patients with Alzheimer type dementia
- Experimental groups: : The subjects were allocated to 3 groups: a group receiving a placebo 3 times a day, a group receiving 30 mg of idebenone 3 times a day, and a group receiving 90 mg of idebenone 3 times a day.
- Method of administration: : Immediately after each meal
- Duration of administration: : 6 months
- Analytical population size: :
- 3-times-a-day placebo group: 100 subjects (31 males and 69 females)
- 3-times-a-day 30 mg idebenone group: 100 subjects (32 males and 68 females)
- 3-times-a-day 90 mg idebenone group: 100 subjects (40 males and 60 females)

There was no bias in background factors or pre-administration values for efficacy evaluation index among the three groups. Efficacy was evaluated by the Alzheimer's Disease Assessment Scale (ADAS) [American Journal of Psychiatry, Vol. 141, pp. 1356-1364, (1984)], an index for rating the therapeutic effect on Alzheimer's disease recommended by the FDA and all over the world. The results were statistically analyzed to rate the efficacy. The results are given in Table 1.

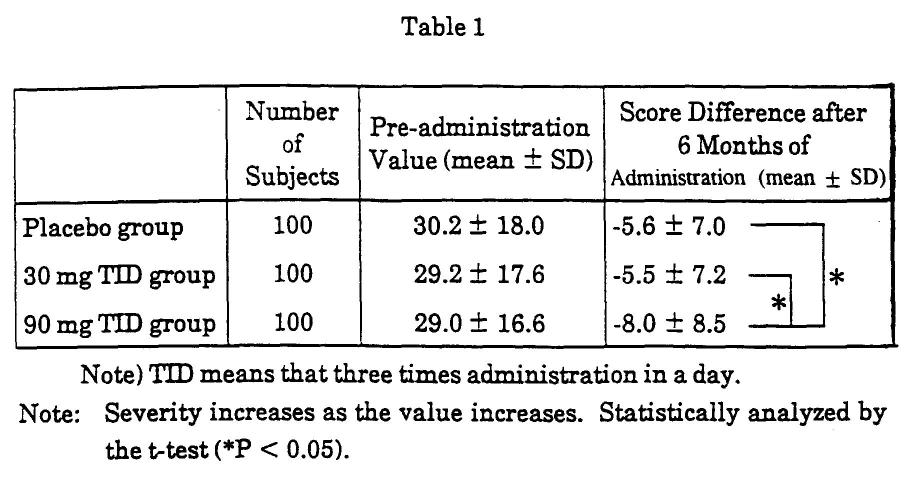

The drugs administered to the patients in the experiment were the tablet preparations of Example 1 and Comparative Examples 1 and 2 above.

In the group receiving 90 mg of idebenone 3 times a day, in comparison with the group receiving 30 mg 3 times a day and the placebo group, various symptoms of Alzheimer's disease showed improvement of statistical significance.

### [Effect of the invention]

With low toxicity, idebenone can be effectively used to treat Alzheimer's disease at daily doses of 270 mg, 360 mg or 450 mg.

## Claims

1. Use of idebenone for the manufacture of a medicament for oral administration for the treatment of Alzheimer's disease comprising a unit dosage of 90 mg or more of idebenone, wherein the amount of idebenone for administration per day is 270 mg.

2. Use of idebenone for the manufacture of a medicament for oral administration for the treatment of Alzheimer's disease comprising a unit dosage of 90 mg or more of idebenone, wherein the amount of idebenone for administration per day is 360 mg.

3. Use of idebenone for the manufacture of a medicament for oral administration for the treatment of Alzheimer's disease comprising a unit dosage of 90 mg or more of idebenone, wherein the amount of idebenone for administration per day is 450 mg.

4. Use according to any of the preceding claims, wherein the medicament is to be administered so that a dose of idebenone per administration is to be administered 3 to 5 times a day at intervals of at least 4 hours.

5. Use according to any of claims 1 to 3, wherein the medicament is to be administered so that a dose of idebenone is to be administered twice a day at an interval of at least 6 hours.

6. Use according to any of the preceding claims, wherein the medicament is to be administered so that each dose of idebenone per administration is to be administered after meals.

7. Use according to any of the preceding claims, wherein the medicament is to be administered in the form of tablet, fine subtilae or capsule.

8. Use according to claim 7, wherein said tablet, fine subtilae or capsule is a sustained-release preparation.

9. Use according to any of claims 1 to 3, wherein the medicament is to be administered in the form of tablet or capsule.

## Patentansprüche

1. Verwendung von Idebenon zur Herstellung eines Medikaments zur oralen Verabreichung zur Behandlung der Alzheimer-Krankheit, umfassend eine Einheitsdosis von 90 mg oder mehr Idebenon, worin die Menge an Idebenon zur Verabreichung pro Tag 270 mg beträgt.

2. Verwendung von Idebenon zur Herstellung eines Medikaments zur oralen Verabreichung zur Behandlung der Alzheimer-Krankheit, umfassend eine Einheitsdosis von 90 mg oder mehr Idebenon, worin die Menge an Idebenon zur Verabreichung pro Tag 360 mg beträgt.

3. Verwendung von Idebenon zur Herstellung eines Medikaments zur oralen Verabreichung zur Behandlung von Alzheimer-Krankheit, umfassend eine Einheitsdosis von 90 mg oder mehr Idebenon, worin die Menge an Idebenon zur Verabreichung pro Tag 450 mg beträgt.

4. Verwendung nach einem der vorangegangenen Ansprüche, worin das Medikament so zu verabreichen ist, daß die Idebenon-Verabreichungsdosis drei- bis fünfmal täglich in Intervallen von zumindest 4 h zu verabreichen ist.

5. Verwendung nach einem der Ansprüche 1 bis 3, worin das Medikament so zu verabreichen ist, daß die Idebenon-Dosis zweimal täglich in einem Intervall von zumindest 6 h zu verabreichen ist.

6. Verwendung nach einem der vorangegangenen Ansprüche, worin das Medikament so zu verabreichen ist, daß jede Idebenon-Verabreichungsdosis nach den Mahlzeiten zu verabreichen ist.

7. Verwendung nach einem der vorangegangenen Ansprüche, worin das Medikament in Form von Tabletten, feinen Subtilae oder Kapseln zu verabreichen ist.

8. Verwendung nach Anspruch 7, wobei es sich bei den Tabletten, den feinen Subtilae oder den Kapseln um Präparate mit verzögerter Freisetzung handelt.

9. Verwendung nach einem der Ansprüche 1 bis 3, worin das Medikament in Tabletten- oder Kapselform zu verabreichen ist.

## Revendications

1. Utilisation d'idébénone pour la fabrication d'un médicament pour administration par voie orale, destiné au traitement de la maladie d'Alzheimer et comprenant une dose unitaire de 90 mg ou plus d'idébénone, utilisation selon laquelle la quantité d'idébénone administrée journellement est de 270 mg.

2. Utilisation d'idébénone pour la fabrication d'un médicament pour administration par voie orale, destiné au traitement de la maladie d'Alzheimer et comprenant une dose unitaire de 90 mg ou plus d'idébénone, utilisation selon laquelle la quantité d'idébénone administrée journellement est de 360 mg.

3. Utilisation d'idébénone pour la fabrication d'un médicament pour administration par voie orale, destiné au traitement de la maladie d'Alzheimer et comprenant une dose unitaire de 90 mg ou plus d'idébénone, utilisation selon laquelle la quantité d'idébénone administrée journellement est de 450 mg.

4. Utilisation selon l'une quelconque des revendications précédentes, selon laquelle le médicament est administré de manière telle que la dose d'idébénone administrée chaque fois le soit 3 à 5 fois par jour à des intervalles d'au moins 4 heures.

5. Utilisation selon l'une quelconque des revendications 1 à 3, selon laquelle le médicament est administré de manière telle que la dose d'idébénone soit administrée deux fois par jour à un intervalle d'au moins 6 heures.

6. Utilisation selon l'une quelconque des revendications précédentes, selon laquelle le médicament est administré de manière telle que la dose d'idébénone administrée chaque fois le soit après les repas.

7. Utilisation selon l'une quelconque des revendications précédentes, selon laquelle le médicament est administré sous forme de comprimés, de languettes ou de capsules.

8. Utilisation selon la revendication 7, selon laquelle lesdits comprimés, languettes ou capsules se trouvent sous forme d'une préparation à libération prolongée.

9. Utilisation selon l'une quelconque des revendications 1 à 3, selon laquelle le médicament est administré sous forme de comprimés ou de capsules.
